# EUROPEAN PATENT APPLICATION

(11) **EP 4 678 183 A1**
(43) Date of publication of application: **14.01.2026**
(21) Application number: 24188324.8
(22) Date of filing: 12.07.2024
(51) Int. Cl.: A61K 39/00, A61K 39/35, A61K 39/39

(54) **TREATMENT OF IMMUNE DISEASES BY ADMINISTRATION OF MRNA FORMULATIONS**

(71) Applicant: Allero Therapeutics B.V., 3029 AK Rotterdam (NL)
(72) Inventor: Pot, Emil, 9742 PZ Groningen (NL); Leenhouts, Cornelis, 9753 KX Haren (NL); Sorensen, Poul, 92190 Meudon (FR)
(74) Representative: Pot, Emil

(57) **Abstract**

The present invention relates to the treatment of autoimmune and allergic diseases by oromucosal administration of a formulation consisting of an optimized combination of a mRNA molecule encoding an antigen for use in inducing immune tolerance to the antigen in a subject, wherein the mucoadhesive carrier is mucosally administered to the subject.

## Description

### Field of the Invention

The present invention relates to the fields of medicine and immunology. The present invention relates to the treatment of immune mediated disorders e.g. autoimmune and allergic diseases by administration of a formulation consisting of a combination of a mRNA molecule encoding an antigen and a tolerizing bacterial particle loaded on or injected in a mucoadhesive carrier.

### State of the Art

The immune system has the task of distinguishing between self and non-self. The mucosal immune system, present along the respiratory, gastrointestinal and genitourinary tracts, has the additional burden of coexisting with an abundance of bacteria and innocuous antigens, such as food, airborne antigens or the commensally bacterial flora. A key feature of the mucosal immune system is its ability to remain tolerant to these antigens while retaining the capacity to repel pathogens effectively. Introduction of antigen systemically, whether by injection or injury, leads to local infiltration of inflammatory cells and specific immunoglobulin production. By contrast, antigens introduced at mucosal surfaces, such as in the oral cavity, gastrointestinal and genitourinary tracts, elicit active inhibition of the immune response to those antigens systemically. The specific induction of these regulated responses by administration of antigen through the mucosal surfaces of the oral cavity and gastrointestinal tract is known as oral tolerance. Oral administration of antigen can lead to systemic unresponsiveness and is an attractive alternative to immunosuppressive medical inventions that have undesirable side-effects (such as steroids). Tolerance inductions via the mucosa have been proposed as a disease modifying treatment strategy against autoimmune, allergic and inflammatory diseases.

Hence, induction of antigen specific-oral tolerance therapy would be an attractive therapeutic approach. Although oral tolerance was first described in 1911, it was not until the later 1970s that investigators started to address the mechanisms involved (Mayer and Shao, Nat Rev Immunol. 2004, 4:407-419). Several mechanisms have been proposed for the development of oral tolerance, ranging from the deletion of antigen-specific T-cells, over immune deviation and induction of anergy to suppression by Tregs (Mucida et al., J Clin Invest. 2005, 115:1923-1933). Targeted and more efficient delivery of molecules for therapeutic and prophylactic applications is a priority for the pharmaceutical industry. Effective strategies should reduce the required dose, increase safety and improve efficacy by focusing molecules at the desired site of action. Mucosal routes of drug and vaccine delivery offer a number of logistical and biological advantages compared with injection. Oromucosal delivery such as via sublingual or buccal routes is particularly attractive as a result of the ease of administration making it very relevant for individuals, e.g. children, who have difficulties swallowing tablets. Furthermore, in the context of immunotherapy, there is strong scientific rationale for the preventive benefits of targeting the developing immune system of children and immunotherapy via the oromucosal route would allow this.

In the context of allergen sublingual immunotherapy (SLIT) considerable progress has been achieved in the last few years regarding our understanding of the physiology of the oral immune system. This strengthened scientific background has confirmed the relevance of the sublingual route to induce antigen-specific tolerance and, furthermore, has pointed out to the interest of targeting oral antigen presenting cells such as dendritic cells (DCs) and Langerhans cells (oLC) to better mobilize allergen-specific regulatory immune mechanisms. It is now well established that SLIT is a safe oromucosal treatment for allergies, which is now broadly recognized as a valid alternative to subcutaneous immunotherapy (SCIT) and more than 20 years of clinical research has confirmed the relevance of the sublingual route to induce long term allergen-specific tolerance and disease modifying effects. The excellent safety profile of SLIT can be explained by the fact that oral tissues contain few pro-inflammatory cells such as mast cells and eosinophils and other danger signals and that the antigen-presenting cells (APCs) involved in the capture, processing of antigens and the subsequent presentation of derived peptides to naive T cells oral APCs exhibit a tolerogenic phenotype. As a consequence the default response initiated by oral APCs to antigens is tolerance allowing for the administration of intact whole antigens without any systemic inflammatory responses unlike the situation when administering via e.g intravenous, intracutaneous or intradermal routes.

A very efficient way of delivery has been shown by the use of oral mucosal patches that are composed of electrospun nanofibers and contain a formulation of an antigen and a bacterial particle (WO2019/081625). However, issues still remain regarding retaining the antigenic property of the antigen from the manufacturing of the patch, applying it to the oral mucosa and throughout its final presentation to the APCs. Therefore, there is still a need to find an alternative antigenic molecule that is more stable and has the properties to efficiently induce an immune tolerance effect.

A possible alternative antigenic molecule could be a mRNA-molecule that encodes an antigen and formulated in an oral patch. However, tis presents several scientific and technical challenges, such as stability, delivery through the oral mucosa and manufacturing challenges. mRNA is inherently unstable and susceptible to degradation by enzymes such as RNases. Protecting mRNA from these degrading enzymes is crucial. Current mRNA vaccines and therapeutics use lipid nanoparticles (LNPs) to encapsulate and protect the mRNA, but these are typically administered via injection. Delivery through the Oral Mucosa: The oral mucosa presents a barrier to drug delivery. For an mRNA oral patch to be effective, it must facilitate the absorption of the mRNA through this barrier into the bloodstream or target cells. This is more challenging than delivering small molecules or proteins. Protection from the Oral Environment: The oral cavity contains saliva and various enzymes that can degrade mRNA. An oral patch would need to provide a protective environment for the mRNA until it is absorbed. Formulation and Manufacturing: Creating a stable, effective formulation that can be manufactured at scale is another hurdle. The formulation must ensure the mRNA's integrity and effectiveness over time.

### Summary of the Invention

While there are significant challenges to formulate mRNA molecules into an oral patch for delivery to the lymph nodes and presentation to the APCs, we have surprisingly found an embodiment where mRNA can both be stabilized in nanofibers as well as being delivered effectively through the oral mucosa to the immune system.

Surprisingly, we found an optimal combination of nanofibers for electrospinning and stabilizing a mRNA molecule encoding an antigen that is causing an immune response in patients suffering from e.g. allergic rhinitis and allergic asthma, multiple sclerosis, type I diabetes, autoimmune uveitis, autoimmune thyroiditis, autoimmune myasthenia gravis, rheumatoid arthritis, pemphigus vulgaris, Sjögren's disease, neuromyelitis optica, food allergy or celiac disease, which is delivered via the oromucosal route when combined with non-living bacterial particles derived from food-grade lactic acid bacteria or probiotic bacteria, inducing an antigen-specific and durable immune tolerance in short course treatments. We observed that the oromucosal administration of such a mRNA molecule by a formulation with non-living bacterial particles incorporated in said mucoadhesive hydrogels or electrospun fibers, composed of an optimal combination of polymers for stabilizing the mRNA molecule, enables slow release, better presentation and extended exposure of the antigen, and gives a significantly better suppression of the antigen-specific immune response in comparison to known delivery methods of said antigen alone or in only combination with said bacterial particles.

We have observed that stabilizing mRNA in electrospun nanofibers requires using polymers that can protect the mRNA from degradation, provide a suitable release profile, and be biocompatible. Here are some polymers that have shown promise in the context of the invention hereunder: Polyethylene Glycol (PEG). Poly(lactic-co-glycolic acid) (PLGA), Polycaprolactone (PCL), Polyvinyl Alcohol (PVA), Chitosan , Poly(lactic acid) (PLA), Polyethyleneimine (PEI) and Gelatin.

### Detailed Description of the Invention

Throughout this disclosure, various publications, patents and published patent specifications are referenced by an identifying citation. The disclosures of these publications, patents and published patent specifications are hereby incorporated by reference.

### General Techniques

The practice of the present invention will employ, unless otherwise indicated, conventional techniques of organic chemistry, pharmacology, molecular biology (including recombinant techniques), cell biology, biochemistry, and immunology, which are within the skill of the art. Such techniques are explained fully in the literature, such as, "Molecular Cloning: A Laboratory Manual" Second Edition (Sambrook et al., 1989); "Oligonucleotide Synthesis" (M. J. Gait, ed., 1984); "Animal Cell Culture" (R. I. Freshney, ed., 1987); the series "Methods in Enzymology" (Academic Press, Inc.); "Handbook of Experimental Immunology" (D. M. Weir & C. C. Blackwell, eds.); "Gene Transfer Vectors for Mammalian Cells" (J. M. Miller & M. P. Calos, eds., 1987); "Current Protocols in Molecular Biology" (F. M. Ausubel et al., eds., 1987, and periodicals) "Polymerase Chain Reaction" (Mullis et al., eds., 1994); and "Current Protocols in Immunology" (J. E. Coligan et al., eds., 1991).

### Definitions

As used herein, certain terms may have the following defined meanings. As used in the specification and claims, the singular forms "a", "an" and "the" include plural references unless the context clearly dictates otherwise. For example, the term "a cell" includes a plurality of cells, including mixtures thereof. Similarly, use of "a compound" for treatment or preparation of medicaments as described herein contemplates using one or more compounds of this invention for such treatment or preparation unless the context clearly dictates otherwise.

As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives, and the like. "Consisting of'" shall mean excluding more than trace elements of other ingredients and substantial method steps for administering the compositions of this invention. Embodiments defined by each of these transition terms are within the scope of this invention.

### Invention

We demonstrate that an optimal combination of nanofibers for electrospinning and stabilizing a mRNA molecule, encoding an antigen that is causing an immune response in patients suffering from e.g. allergic rhinitis and allergic asthma, multiple sclerosis, type I diabetes, autoimmune uveitis, autoimmune thyroiditis, autoimmune myasthenia gravis, rheumatoid arthritis, pemphigus vulgaris, Sjögren's disease, neuromyelitis optica, food allergy or celiac disease, which is delivered via the oromucosal route when combined with non-living bacterial particles derived from food-grade lactic acid bacteria or probiotic bacteria, is inducing an antigen-specific and durable immune tolerance in short course treatments. We observed that the oromucosal administration of such a mRNA molecule by a formulation with non-living bacterial particles incorporated in said mucoadhesive hydrogels or electrospun fibers, composed of an optimal combination of polymers for stabilizing the mRNA molecule, enables slow release, better presentation and extended exposure of the antigen, and gives a significantly better suppression of the antigen-specific immune response in comparison to known delivery methods of said antigen alone or in only combination with said bacterial particles.

We have observed that stabilizing mRNA in electrospun nanofibers requires using polymers that can protect the mRNA from degradation, provide a suitable release profile, and be biocompatible. Here are some polymers that have shown promise in the context of the invention hereunder: Polyethylene Glycol (PEG). Poly(lactic-co-glycolic acid) (PLGA), Polycaprolactone (PCL), Polyvinyl Alcohol (PVA), Chitosan , Poly(lactic acid) (PLA), Polyethyleneimine (PEI) and Gelatin.

Oromucosal administration of a mRN molecule encoding an immune dominant antigen presented and formulated with bacterial particles in a mucoadhesive patch, film or hydrogel comprised of nanofibres induces superior suppression of local and systemic T-cell responses. Treatment resulted in an antigen-specific decrease of the proliferative capacity of the splenocytes and lymph node cells, which was critically dependent on the production of IL-10 and TGF-β and associated with a significant induction of *Foxp3*+ regulatory T-cells. This approach of antigen-formulated bacterial particals has the capacity for potentiating oral tolerance even in the setting of established hypersensitivity. Thus it is applicable for the treatment of celiac disease and other autoimmune and/or allergic diseases. The efficacy of the invention was demonstrated in autoimmune and allergic disease animal models, as well as in the context of human immune disorders derived *ex vivo* models.

The goal of the invention is achieved by means of specific polymers or polymer combinations that is used for the electrospun mucoadhesive patch, film or hydrogel. These polymers and polymer combinations are protecting the mRNA molecule from the Oral Environment: The oral cavity contains saliva and various enzymes that can degrade mRNA. An oral patch would need to provide a protective environment for the mRNA until it is absorbed. Formulation and Manufacturing: Creating a stable, effective formulation that can be manufactured at scale is another hurdle. The formulation must ensure the mRNA's integrity and effectiveness over time.

In this way, the overall solubility and rate of dissolution of such antigen containing substance is made optimal by techniques know in the art in order to increase their bioavailability. The effect is most significant in variants, by which the antigen containing substance is incorporated directly in the material of the nanofibers, such as for example disclosed in WO 2014/131376 or electrohydrodynamically obtained fibres, such as for example disclosed in WO2015/189212 and to be formulated into a mucoadhesive patch, including but not limited to such mucoadhesive patch as disclosed in WO2012/097763 and WO2016/051159. In a preferred embodiment, the mucoadhesive patch is a patch as disclosed in WO2016/051159. In another embodiment of the invention, the antigen containing substance is formulated into bioadhesive films, such as for example in US2015125495 and WO2016/079246. In a further embodiment of the invention, the antigen containing substance is formulated into hydrogel matrices such as for example disclosed in "Advances in Physicochemical Properties of Biopolymers" Part 2 (Mansuelli & Renard, eds., 2017, eISBN: 978-1-68108-544-9), and preferably a biodegradable mucoadhesive hydrogel.

The mRNA molecule as used in the invention hereunder, is a single-stranded nucleic acid that carries genetic information from DNA to the ribosome, where it serves as a template for the antigen of the invention hereunder. The features of the mRNA molecule of the invention is a single-stranded nucleic acid, allowing it to be read by the ribosome, that might have a modified guanine nucleotide at the 5' end and a coding sequence that is translated into an antigen of the invention, further containing specific codons that signal the beginning (start codon, typically AUG) and end (stop codons, such as UAA, UAG, or UGA) of the antigen-coding sequence and a polyadenylation (Poly-A) tail of a string of adenine nucleotides (the poly-A tail). Finally the mRNA molecule may contain untranslated rflanking the coding sequence, known as the 5' UTR and 3' UTR, which play roles in the regulation of mRNA stability, localization, and translation efficiency. The mRNA molecule may be a synthetic mRNA molecule to be designed to produce specific antigens of the cell.

We have observed that the polymers and polymer combination of the invention is protecting the mRNA molecule from nucleases and other degrading enzymes. Also, the polymer nanofibers of the invention can be engineered to release mRNA in a controlled manner. This can be achieved by modifying the composition and structure of the nanofibers, allowing for sustained release of the mRNA over time. An important feature of the invention is that these polymer nanofibers are electrospun nanofibers, which involves using an electric field to draw out fibers from a polymer solution, which encapsulate the mRNA molecule. The properties of the resulting nanofibers can be tuned by adjusting the polymer composition and electrospinning parameters.

Stabilizing mRNA molecules in electrospun nanofibers requires using polymers that can protect the mRNA from degradation, provide a suitable release profile, and be biocompatible. Here are some polymers that have shown promise in the context of our invention:
- Polyethylene Glycol (PEG): PEG is a hydrophilic polymer known for its ability to increase stability and reduce immunogenicity. PEG can form nanofibers that protect mRNA from degradation and improve its bioavailability.
- Poly(lactic-co-glycolic acid) (PLGA): PLGA is a biodegradable and biocompatible polymer widely used in drug delivery. It can form nanofibers that encapsulate and protect mRNA, providing controlled release through hydrolytic degradation.
- Polycaprolactone (PCL): PCL is another biodegradable polymer that can be used to create nanofibers. It has a slower degradation rate compared to PLGA, which can be useful for sustained release applications.
- Polyvinyl Alcohol (PVA): PVA is a water-soluble, biocompatible polymer that can form stable nanofibers. It can protect mRNA from degradation and allow for controlled release when crosslinked or blended with other polymers.
- Chitosan: Chitosan is a natural polysaccharide with mucoadhesive properties and inherent antimicrobial activity. It can form nanofibers that protect mRNA and facilitate its uptake by cells. Chitosan can be used alone or in combination with other polymers to improve stability and delivery efficiency.
- Poly(lactic acid) (PLA): PLA is a biodegradable polymer that can form nanofibers with good mechanical properties and stability. PLA nanofibers can protect mRNA and provide controlled release through hydrolytic degradation.
- Polyethyleneimine (PEI): PEI is a cationic polymer that can form complexes with mRNA, protecting it from degradation and facilitating cellular uptake. PEI can be used to form nanofibers that enhance mRNA stability and delivery efficiency.
- Gelatin: Gelatin is a natural polymer that can form biocompatible and biodegradable nanofibers. It can encapsulate mRNA, protect it from degradation, and release it in a controlled manner.

### Combination of Polymers

An important feature of the invention are the combination of polymers that have shown to achieve the desired properties for mRNA stabilization and delivery, such as:
- PEG-PLGA: Combining PEG and PLGA to create nanofibers with improved hydrophilicity, stability, and controlled release profiles.
- PVA-Chitosan: Blending PVA with chitosan to enhance the mechanical strength, stability, and mucoadhesive properties of the nanofibers.

But also other specific combinations of suitable polymers as mentioned above have shown the desired properties to effectively deliver the mRNA molecules to the immune system. By carefully selecting and combining these polymers and functionalizing the nanofibers, it is possible to create a delivery system that stabilizes mRNA molecules and enhances its therapeutic potential.

In one embodiment, the polymer layers can also comprise plastisizers, such as polyols, phtalhates or citrates to enhance the plasticity of the layers, and/or absorption accelerators, such as acetylcysteine, surface active substances, chelating substances, fatty acids, polyols, dextran sulphates, sulfoxides, Azone^{®}, (lyso)phosphatidylcholine, metoxysalicylate, menthol, aprotonin, dextran sulphate, cyclodextrins, 23-lauryl ether to facilitate the penetration of particles.

In a first aspect the invention relates to a mucoadhesive carrier comprising a non-living bacterial particle and a mRNA molecule encoding an antigen for use in inducing immune tolerance to the antigen in a subject, wherein the mucoadhesive carrier is mucosally administered to the subject. Mucosal administration of the mucoadhesive carrier is understood to comprise contacting the mucoadhesive carrier to a mucous membrane or mucosa of the subject. It is further understood herein that induction of immune tolerance is herein understood to comprise increasing or enhancing an already existing (too weak) tolerance.

Preferably the invention relates to a method for inducing immune tolerance to an antigen, comprising oromucosal administration of said antigen by a bacterial particle-mucoadhesive patch, film or hydrogel formulation.

The terms "immune tolerance", "immunological tolerance', "tolerance' or "desensitise' are here defined as to make a sensitised or hypersensitive subject, less sensitive, insensitive or nonreactive to a given antigen for which the subject is to be tolerized by reducing the immunological reactivity of a subject towards the antigen. Immune tolerance may be generated, for example, by exposure of mucosal surfaces to tolerance-inducing antigen-BLP comprising mucoadhesive carrier compositions as defined herein. Mucosal administration of both high- and low-dose antigen may result in immune tolerance, in which the immune response to subsequent systemic administration of antigen is reduced. At least two mechanisms of immune tolerance may exist. Tolerance to high-doses of an antigen appears to occur by inactivation or clonal deletion of Th1 and Th2 cells. In contrast, tolerance to low doses of antigen leads to bystander immune Suppression mediated by stimulation of Treg cells to produce Suppressive cytokines Such as interleukin-4 (IL-4), interleukin-10 (IL-10) and TGF-β. See also Anderson and Jabri (Current Opinion in Immunology 2013, 25:410-417) and Rayner and Isaacs (Seminars in Arthritis & Rheumatism (2018), doi:

### https://doi.orq/10.1016/i.semarthrit.2018.09.008).

Preferably the invention relates to the use of a bacterial partical-mucoadhesive patch, film or hydrogel formulation of an antigen for the preparation of a medicament, medical food or nutraceutical for oromucosal administration to treat an immune response related disease in a patient, wherein said antigen is released at the oromucosal tissue of said patient.

Preferably, said antigen is delivered by an antigen formulated in a bacterial particle-mucoadhesive patch, film or hydrogel.

Preferably, the present invention relates to the use of an antigen formulated with bacterial particles for the preparation of a medicament for oromucosal administration to induce immune tolerance.

Preferably, said immune tolerance is induced in a patient. Said patient is preferably a human or an animal. Said animal is preferably a mammal, and preferably chosen from the group consisting of mice, rats, rabbits, cats, dogs, pigs, cows, sheep, and horses. Preferably, said mammal is human. Preferably, said immune tolerance is mucosal tolerance.

### Mucosa

*Mucosa* as used here can be any mucosa such as oral mucosa, rectal mucosa, urethral mucosa, vaginal mucosa, ocular mucosa, pulmonary mucosa and nasal mucosa.

*Oromucosal administration* as used throughout the application encompasses the targeted delivery to the oral mucosa. Oromucosal administration includes buccal, sublingual and gingival routes of delivery. Preferably, said oromucosal delivery is buccal or gingiva administration and said tolerance is oral tolerance.

*Oromucosal tolerance* as used here throughout the application is the inhibition of specific immune responsiveness to an antigen in an animal (including humans), after said animal has been exposed to said antigen via the oromucosal route. Preferably, said mucosal tolerance is systemic tolerance.

The present invention thus relates to a method or use as described herein, wherein said induction of immune tolerance is more effective at least 1.5, preferably 2, more preferably 3 times or more relative to before said induction. Alternatively, said antigen is better tolerated at least 1.5, 2, 3 times or more relative to before said induction. The induction of immune tolerance can be measured by methods known in the art. Preferably, said induction of immune tolerance can be measured by modulation of a cytokine level in said animal. As such, the modulation can be an increase of a cytokine level, for instance said increase of a cytokine level is at least 1.5, 2, 3 times or more relative to before said induction, e.g. IL-10 or TGF-β. Alternatively, said modulation is a decrease of the level of a particular cytokine level, for instance said decrease of the cytokine level is at least 1.5, 2, 3 times or more relative to before said induction, e.g. IL-12, IL-17 and IFN-γ. The cytokines which are modulated may be chosen from any relevant cytokines, preferably said cytokines are chosen from the group consisting of IL-2, IL-4, IL-5, IL-6, IL-8, IL-10, IL-12, IL-13, IL-17, IL-23, TNF-α, IFN-γ, IFN-α, MCP-1, TGF-β, RANK-L and Flt3L.

### Tolerizing compounds

In the present invention, the non-living bacterial particles stimulate the immune system to enhance antigen-specific tolerogenic immune responses, and at the same time may deliver the antigen at the intended site, e.g. the oral mucosa.

Tolerogenic compositions containing non-living bacterial particals according to the present invention may be prepared, tested for immunogenicity, efficacy and safety employing the technology disclosed in published PCT application WO 02/101026, hereby incorporated by reference.

Preferably, the non-living bacterial particle for use in accordance with the invention is derived from a probiotic bacterium. More preferably the non-living bacterial particle is derived from a (probiotic) *Lactobacillius,* a *Lactococcus,* a *Bifidobacterium* or an *Escherichia coli* Nissle. Most the non-living bacterial particle for use in accordance with the invention is derived from at leat one of *Lactococcus lactis* MG1363, *Lactobacillus rhamnosus* GG and *Escherichia coli* Nissle 1917.

mRNA containing formulations may be based on particles derived from inactivated *Lactococcus lactis* bacteria, a safe bacterium traditionally used in the food industry, such as for the production of cheese (the particles are elsewhere described as Gram-positive Enhancer Matrix or Bacterium-Like Particles and are herein referred to as "BLPs"). BLPs activate the innate immune systems through Toll-like receptor 2 (TLR2; Ramirez et al., Mucosal Immunology 2010, 3:159-171). TLR2 exerts downstream effects on *myd88.* Recently it was shown that TLR2 is essential for microbiota-induced down-regulation of *myd88* transcription (Koch et al., Nature Comm 2018, 9:4099). The latter being associated with control of inflammatory signalling. Therefore, commensal bacteria in general such as lactic acid bacteria, and BLPs in particular, may constitute suitable tolerogenic compounds. BLPs are obtained by the acidic heat treatment of *L. lactis* bacteria or any other Gram-positive bacterium, resulting in non-living bacterial particles that predominantly consist of a peptidoglycan surface and lack recombinant DNA. The preparation of BLPs is disclosed in WO 02/101026. The antigenic polypeptides of the present invention may be simply mixed with BLPs or may be loaded onto the BLPs, which employs the non-covalent coupling technology referred to as, Protan technology, disclosed in WO 02/101026 and WO2012/128628. The resulting antigen-associated BLPs (antigen mixed with or bound to BLPs) constitute part of therapeutic formulations that may be delivered to humans via the mucosal layers of the oral cavity and other parts of the gastro-intestinal tract (e.g. mucoadhesive patch, mucoadhesive gel, capsule, tablet or liquid), without the need for an injection. Preferrably, the therapeutic formulations with BLPs and antigen are delivered to humans via the mucosal layers of the oral cavity using a mucoadhesive patch, film or hydrogel. Even more preferably, the therapeutic formulations with BLPs and antigen contain additional tolerizing compounds known in the art such as cytotoxic T lymphocyte antigen 4-immunoglobulin G (Maazi et al. 2013, Clin Exp Immunol 172:113-120), zebularine (WO2008/147283), retinoic acid (Coombes et al. 2007, J Exp Med 204:1757-1764), rapamycin (Maldonado and von Adrian 2010, Adv Immunol 108:111-165), vitamin D3 (Taher et al. 2008, J Immunol 180: 5211-5221) and/or D-mannose (Zhang et al. 2017, Nature Med 23:1036-1047).

In another embodiment of the invention, tolerogenic compositions containing non-living bacterial particals may be prepared from empty bacterial envelopes, so-called bacterial ghosts (BGs). Antigen containing formulations may be based on BGs derived from the strain *Escherichia c*oli Nissle 1917, a safe bacterium used in the the probiotic pharmaceutical product Mutaflor^{®} or any other safe Gram-negative bacterium. BGs are produced by controlled heterologous expression of a gene which causes a partial lysis of the cell membrane, as disclosed in WO0053163 and WO2015124717, hereby incorporated by reference. An example of such a lytic gene is the gene E of the bacteriophage PhiX174 which codes for a polypeptide which is inserted into the cell membrane complex of Gram-negative bacteria and when oligomerized leads to the formation of a transmembrane tunnel structure through the inner and outer membrane. The inner diameter of this tunnel structure can be 40 to 200 nm or 500 to 1000 nm depending on the lysis conditions. The cytoplasmic material of the cell is released through this tunnel and leaves behind an empty cell envelope having an intact morphology, which can serve as well as delivery vehicle since the empty cell envelopes can be loaded with antigens (WO0053163, WO0154672, WO2005011713). The antigenic polypeptides of the present invention may be simply mixed with BGs or they may be loaded into the empty cell envelopes or they may be co-expressed with the bacteriophage lysin and anchored to the cell envelopes by techniques known in the art (WO9906567; WO0044878). In addition, BGs can also be prepared from Gram-positive bacteria by using a chimeric E-L lysis gene (US 5,075,223).

BGs are exceptionally suitable as carriers or targeting vehicles for antigens or antigen containing material. Since the BGs only contain the desired antigens, a high degree of loading and thus a high tolerizing efficiency of the antigen can be achieved. Notwithstanding the carrier option for BGs, the tolerizing effect can also be achieved by simply mixing the antigen with the BGs. Overall, bacterial ghosts are a safe tolerizing material since they are non-living bacterial particles that do not contain anylonger recombinant DNA (WO03006630, WO2009090093). The resulting antigen-associated BGs (antigen mixed with, loaded into or bound to BGs) constitute part of therapeutic formulations that may be delivered to humans via the mucosal layers of the oral cavity or other parts of the gastro-intestinal tract (e.g. mucoadhesive patch, mucoadhesive gel, capsule, tablet or liquid), without the need for an injection. Preferrably, the therapeutic formulations with BGs and antigen are delivered to humans via the mucosal layers of the oral cavity using a mucoadhesive patch, film or hydrogel. Even more preferably, the therapeutic formulations with BGs and antigen contain additional tolerizing compounds known in the art such as cytotoxic T lymphocyte antigen 4-immunoglobulin G (Maazi et al. 2013, Clin Exp Immunol 172:113-120), zebularine (WO2008/147283), retinoic acid (Coombes et al. 2007, J Exp Med 204:1757-1764), rapamycin (Maldonado and von Adrian 2010, Adv Immunol 108:111-165), vitamin D3 (Taher et al. 2008, J Immunol 180: 5211-5221) and/or D-mannose (Zhang et al. 2017, Nature Med 23:1036-1047).

In a further embodiment of the invention, tolerogenic compositions containing non-living bacterial particals may be prepared from chemically sterilized non-recombinant food-grade lactic acid bacterial - or probiotic bacterial cultures. Antigen-containing formulations may be based on non-living particles derived from chemically sterilized food-grade Gram-positive and Gram-negative bacteria such as, but not limited to, *Lactococcus, Lactobacillus or Escherichia* bacteria. In a preferred embodiment of the invention, chemically sterilized *Lactococcus lactis, Lactobacillus rhamnosus* or *Escherichia coli* Nissle 1917 are used. Chemical sterilization of bacteria is known in the art. In a preferred embodiment of the invention, chemical sterilization is achieved by the use of alcohol solutions. Examples of alcohol solutions are ethanol and isopropyl alcohol, particularly solutions between 60% and 90% alcohol (v/v) and 10 - 40% purified water (v/v), are rapidly antimicrobial against bacteria. A contact time between the bacteria and the alcohol solution of 12 hours or more is preferred in order to achieve complete sterilization.

In another preferred embodiment, chemical sterilization is achieved by the use of betapropiolactone. In the used method, betapropiolactone is preferably added at a final concentration of 0.01% - 0.1% (v/v) and more preferably of 0.025% - 0.5% (v/v). Betapropiolactone may be added in one or more steps, e.g. in two consecutive steps to the preparation, wherein the two portions are preferably of equal amount, wherein the second portion is added about 15-45 min, e.g. at about 30 min. The addition of beta propiolactone preferably occurs as a liquid. Addition as a vapor or aerosol or in other forms is possible. A preferred aspect of the bacterial sterilization by betapropiolactone is an incubation period for 10-60 min, more preferably for 15-45 min, even more preferably for 25-35 min as described in WO2009090093.

Furthermore, suitable incubation temperatures during the chemical sterilization process are between 2°C and 55°C, more preferably between 20°C and 40°C. The alcohols or betapropriolactone are aseptically removed from the inactivated bacterial particles by extensive washing with purified water or pharmaceutically accepted neutral buffers with a pH between 6 and 8, more preferably between pH 6.5 and 7.5. Washing of the bacterial particles is done with at least ten times the incubation volume to efficiently remove the sterilizing agent.

The resulting chemically inactivated bacterial particles are a safe tolerizing material since they are of food-grade origin and non-living. According to the invention the antigen is mixed with the chemically inactivated bacterial particles and constitute part of therapeutic formulations that may be delivered to humans via the mucosal layers of the oral cavity or other parts of the gastro-intestinal tract (e.g. mucoadhesive patch, mucoadhesive gel, capsule, tablet or liquid), without the need for an injection. Preferrably, the therapeutic formulations with chemically inactivated bacterial particles and antigen are delivered to humans via the mucosal layers of the oral cavity using a mucoadhesive patch, film or hydrogel. Even more preferably, the therapeutic formulations with chemically inactivated bacterial particles and antigen contain additional tolerizing compounds known in the art such as cytotoxic T lymphocyte antigen 4-immunoglobulin G (Maazi et al. 2013, Clin Exp Immunol 172:113-120), zebularine (WO2008/147283), retinoic acid (Coombes et al. 2007, J Exp Med 204:1757-1764), rapamycin (Maldonado and von Adrian 2010, Adv Immunol 108:111-165), vitamin D3 (Taher et al. 2008, J Immunol 180: 5211-5221) and/or D-mannose (Zhang et al. 2017, Nature Med 23:1036-1047).

### Antigens

The sequence encoding the antigen can be obtained from any natural source and/or can be prepared synthetically using well-known DNA synthesis techniques. The sequence encoding the antigen can then (for instance) be incorporated in a suitable expression vector, which is then used to transform or transfect the intended host. The recombinant host cell thus obtained can then be cultured, upon which the isolated antigen can be used to formulate the therapeutic composition, optionally after further purification and/or processing steps, such as freeze-drying to form a powder. Optionally, the recombinant host cell bacteria is used to produce BGs, in which case the antigen constitutes part of the BGs and protein purification steps are not required, but instead the BGs containing the antigen of interest are used directly to formulate the therapeutic composition. Antigens can also be produced using well-known protein chemistry techniques. Furthermore, antigens can be obtained as whole intact antigens or partial antigens from extracts, such as pollen, gluten, house dust mite, cat saliva, birch or peanut.

An antigen can be any antigen known to the person skilled in the art. An antigen as used here throughout the application is preferably any substance that provokes an immune response when introduced in the body of an animal, wherein said immune response can be T-cell mediated and/or a B-cell mediated response. The antigen may comprise a T-cell epitope and/or a B-cell epitope. The length of the antigen is not particularly limiting, provided said antigen can be produced. In a preferred embodiment of the invention, the antigen is a whole intact antigen. The antigen can be a protein or a part thereof, such as a polypeptide or a peptide. The antigens according to the invention include linear and/or conformational epitopes. T-cell mediated responses cover Th1, Th2 and/or Th17 responses. The antigen can be any antigen, such as, but not limited to allergens (including food allergens), allo-antigens, self-antigens, auto-antigens, and therapeutic molecules or antigens that induce an immune response. Preferably, said antigen is involved in the induction of immune response related diseases. Even more preferably, said antigen is involved in the induction of allergic rhinitis, allergic asthma, multiple sclerosis, type I diabetes, autoimmune uveitis, autoimmune thyroiditis, autoimmune myasthenia gravis, rheumatoid arthritis, food allergy, celiac disease, pemphigus vulgaris, neuromyelitis optica, graft versus host disease or anti-drug antibody (ADA) development. Examples of antigens as part of the invention include, but are not limited to: gliadin, hordein, Der p 1, Der p 2, Der p 2.1, Fel d1, pINS, GAD65, InsB₉₋₂₃ , MBP, Dsg3, aquaporin 4 or IA2. A more extended list of antigens as part of the invention is found in the Table 8.

The inventors observed that the delivery via a mucoadhesive carrier of immunodominant antigens combined with bacterial particles of the invention suppress systemic inflammatory T-cell responses, and that such formulation and delivery of antigens are necessary and sufficient for the induction of a significant tolerogenic effect.

Regulatory T cells (Treg) play a critical role in the induction and maintenance of oral tolerance. Induction of Treg is a major goal for immunotherapy for several autoimmune, allergic and inflammatory diseases. Current strategies for therapeutic induction of antigen-specific suppressor cells face significant hurdles, and usually require strenuous techniques to isolate, handle and transfer adequate numbers of regulatory cells. The bacterial particle-antigen mucoadhesive carrier delivery system of the present invention circumvents these problems and effectively induces antigen-specific Treg. In the present invention, it was demonstrated that induction of Treg can be achieved by exposing the mucosal immune system to low doses of antigen. The exposure to low doses of antigen is preferably a continued exposure. Hence, the present invention relates to antigens inducing and/or expanding Treg cells, preferably CD4⁺CD25⁺, CD4⁺CD25⁻ and CD8⁺ Treg cells.

It was further demonstrated in the present invention that the Treg cells which were induced and/or expanded by the antigens according to the invention function through a TGF-β and/or IL-10 dependent mechanism. Previously evidence has been provided that TGF-β plays a critical role in oral tolerance as well as in the development of peripheral induced Treg. Accordingly, the present invention provides immunodominant antigens which stimulate endogenous TGF-β and/or IL-10 expression.

Moreover, it was shown that antigen-specific TGF-β producing Th3 cells drive the differentiation of antigen-specific *Foxp3*⁺ regulatory cells in the periphery. Furthermore, TGF-β dependent conversion of peripheral CD4⁺CD25⁻ T cells into CD25⁺, CD45RB^{-/low} suppressor cells has been reported. It was shown that oral tolerance induced by CTB-conjugated Ag is associated with increase in TGF-β by the generation of both *Foxp3*⁺CD25⁺ and both *Foxp3*⁺ and *Foxp3*⁻CD25⁻CD4⁺ regulatory T cells. These data suggest a key role for *Foxp3*⁺ 'adaptive' Treg in the induction and maintenance of oral tolerance. We also show a significant mucosal *Foxp3* induction. Moreover, the 'mucosal' induced regulatory T-cell tends to be antigen specific as bacterial particles alone are unable to induce this *Foxp3* upregulation within the GALT. Accordingly, the present invention relates preferably to Foxp3+ Treg cells.

The present invention further demonstrated that the Treg cells which were induced and/or expanded by the antigens according to the invention decreased inflammation, in particular in the spleen and secondary lymph node cells. Moreover, the IFN-γ and IL-12 production was decreased. Accordingly, the present invention provides immunodominant antigens which decrease endogenous IFN-γ and/or IL-12 production, and/or stimulate endogenous TGF-β and/or IL-10 expression. Moreover, the present invention relates to antigens reducing proliferation of spleen and/or lymph node cells. It will be appreciated that the present invention relates also to antigens suppressing inflammatory antigen-specific T cell responses.

### Immune response

An immune response related disease as used here is a disease caused by an unwanted immune response of the body against an antigen, whereby said antigen can be either a heterologous antigen or an auto-antigen. Preferably in the methods of the invention for inducing immune tolerance to an antigen, the antigen causes or is associated with an immune response related disease. Immune response related diseases include, but are not limited to allergic reaction including food allergy, celiac disease, allergic asthma, allergic rhinitis, allergic conjunctivitis, atopic dermatitis, autoimmune uveitis, autoimmune thyroiditis, autoimmune myasthenia gravis, rheumatoid arthritis, type I diabetes, pemphigus vulgaris and multiple sclerosis. Immune response related diseases also include unwanted immune reactions such as graft versus host disease or immune activation of medication such as the antibody production against non endogenous Factor VIII. Preferably, the disease is selected from the group consisting of allergic rhinitis, allergic asthma, food allergy, celiac disease, type I diabetes and immune inactivation of therapeutics.

In one embodiment, the invention pertains to a mucoadhesive carrier comprising a non-living bacterial particle and an antigen as herein defined for use in the prevention and/or treatment of an immune response related disease as herein defined, wherein the mucoadhesive carrier is mucosally administered to the subject as herein defined, and wherein preferably, the antigen causes or is associated with the immune response related disease.

According to the present invention the term "immunodominant" relates to the principle of antigens, including peptide fragments and specific epitopes thereof, inducing an immune response.

In view of the above, it will thus be appreciated that the present invention relates to method or use as described herein, wherein said method or use is therapeutic and/or prophylactic.

A further aspect of the invention relates to a method for inducing immune tolerance to an antigen, including peptide fragments and specific epitopes thereof, comprising oromucosal administration of said antigen in combination with bacterial particles in a mucoadhesive patch, film or hydrogel. The antigen may be produced by the micro-organism that is used to make the bacterial particles. In a preferred embodiment of the invention, the purified antigen is produced from another source and mixed with the bacterial particles. The antigen may be physically attached to the tolerizing bacterial particle compound, but the mixed mode can also be without physical interaction of the bacterial particles and the antigen.

### Medicament and administration

*Compound* means any chemical or biological compound or complex, including simple or complex organic and inorganic molecules, peptides, peptido-mimetics, proteins, protein complexes, antibodies, carbohydrates, nucleic acids or derivatives thereof. An immune-modulating compound is a compound that modifies the function of the immune system. An immune-modulating compound as used here is a tolerance inducing compound; tolerance induction can be obtained, as a nonlimiting example, in a direct way by inducing regulatory T-cells such as Treg, Tr1 or Th3, or by shifting the Th1/Th2 balance towards Th1 or Th2, or by inhibiting Th17, or in an indirect way, by activation of immature dendritic cells to tolerizing dendritic cells and/or inhibiting Th2 immune response inducing expression of "co-stimulation" factors on mature dendritic cells. Immune-modulating and immune-suppressing compounds are known to the person skilled in the art and include, but are not limited to organic molecule such as vitamin D3 (or its precursors), D-mannose zebuline, retinoic acid and phosphatidylserine, bacterial metabolites such as spergualin, fungal and streptomycal metabolites such as tacrolimus, rapamicin or ciclosporin, immune-suppressing cytokines such as IL-4, IL-10, IFNα, TGFβ (as selective adjuvant for regulatory T-cells), Flt3L, TSLP and Rank-L (as selective tolerogenic DC inducers), antibodies and/or antagonist such as anti-CD40L, anti-CD25, anti-CD20, anti-IgE, anti-CD3, anti-IL-6 (or IL6R) and proteins, peptides or fusion proteins such as the CTL-4 Ig or CTLA-4 agonist fusion protein.

*Delivery* or *Administration* as used here means any method of delivery or administration via mucoadhesive materials known to the person skilled in the art and includes, but is not limited to patches, biofilms or hydrogels comprising or carrying the antigens or bacterial particles combined with the antigens, optionally in presence of compounds such as Vitamine D3 and/or D-mannose that may further enhance the tolerogenic effect and/or penetration enhancers such as surface active components or chelating agents that may enhance oromucosal administration and/or mucosal uptake.

*Hydrogels* or films as used here mean water swellable, cross-linked polymers that can be impregnated or loaded with antigens and bacterial particles. The bacterial particle-antigen combination loaded into the hydrogel is released in a controlled manner as the hydrogel becomes hydrated within the oral cavity. In a preferred embodiment, the hydrogel matrix in the present invention is comprised of polysaccharides such as chitosan, alginate, and hyaluronic acid. In a more preferred embodiment of the invention the hydrogel matrix is composed of chitosan. Chitosan is a bioactive, biocompatible, biodegradable non-toxic compound with favourable properties for a range of industrial and biomedical applications, including drug delivery, wound healing and biomedical implants. Chitosan is a polysaccharide comprising 1-4-linked residues of 2-mino-2-deoxy-beta-D-glucose (glucosamine) and 2-acetamido-2-deoxy-beta-D-glucose (N-acetylglucosamine). Chitosan is prepared by at least partial deacetylation of the naturally occurring polysaccharide chitin (poly-N-acetylglucosamine or (1→4)-2-deoxy-beta-D-glucan), which is found naturally in the shells of crustaceans, insects and fungi. Thus acetyl groups are removed from at least some of the N-acetylglucosamine residues of chitin to form glucosamine residues.

In commercial preparations of chitosan, usually from about 50% to about 100% of the N-acetylglucosamine residues of chitin have been deacetylated to glucosamine residues. In the present invention chitosan with a deacetylation percentage of 70% to 95% is preferred. Chitosan dissolves to a significant extent in acidic solution, values below pH 6.5. Thus, soluble chitosan is cationic, allowing it to bind to negatively charged surfaces and biological materials. Chitosan is a prominent example of a polysaccharide that can be crosslinked ionically. Chitosan hydrogels can be physically mixed into stable networks by introducing anionic ions or macromolecules to neutralize the positively charged chitosan and induce electrostatic attraction within the gelatinized network. Ionic crosslinking is a relatively safe technique to use for fabricating biocompatible hydrogels without toxic catalysts. According to the present invention chitosan-based hydrogels formulated with non-living bacterial particles and antigen can be dried for long-term storage and easy application in the oral cavity.

In another preferred embodiment of the invention a thermosensitive chitosan solution is used. To obtain a thermosensitive hydrogel, a chitosan solution is neutralized with a polyol counterionic monohead salt is used to neutralize the chitosan solution. Under these conditions chitosan remains liquid at or below 25°C and can be stored for a long time without losing the thermosensitive properties. The system can then have a pH value within a physiologically acceptable neutral range (pH 6.8-7.2) and it is only the temperature of the milieu that determines the liquid or gel state, gel formation being observed upon an increase in temperature. In a preferred embodiment of the invention the polyol counterionic monohead salt is beta-glycerolphospate and gel formation starts at temperature above 32°C. The production and use of thermosensitive chitosan-based hydrogels is well described in literature, such as "Biological Activities and Application of Marine Polysaccharides" (Argüelles-Monal et al., eds).

Mucoadhesive patches as used here mean patches that consists of multiple polymer layers with different functions. In such multi-layer patches, a mucoadhesive layer covers and overlaps a nanoscaffold reservoir layer as decribed in Masek et al. (2017, J Control Rel 249:183-195). The mucoadhesive layer serves to attach/adhere to the oral mucosa and is optionally covered by a non-adhesive backing layer to prevent diffusion of the medication into the oral cavity. The nanoscaffold reservoir layer is the actual carrier of the medicament and faces the oral mucosa. The manufacturing of this type of mucoadhesive patch that may involve electrospinning of the nanofibers in the reservoir layer is described in detail in WO2016/051159. In a preferred embodiment therefore, the mucoadhesive (e.g. particle) carrier, comprises: a) a nanoscaffold (or matrix) carrying or comprising at least one substance or API (e.g. in the form of particles), and b) a mucoadhesive (layer), wherein the mucoadhesive (layer), on at least a part of its surface, can adhere (to a mucosa) or overlap with the nanoscaffold. The nanoscaffold preferably contains or has pores having the size of from 10 nm to 1,000 µm and/or is a nanofibrous layer of a thickness in the range 0.1 to 1,000 µm; or comprises a layer of biocompatible polymers or a mixture thereof. 3. The mucoadhesive carrier according to this embodiment can be further characterized in that: a) the mucoadhesive layer (at least partially) overlaps the nanoscaffold, an edge of the mucoadhesive layer overlaps an edge of the nanoscaffold and/or the mucoadhesive layer surrounds the nanoscaffold along an edge; or b) it is adapted for application onto a target mucosa, the nanoscaffold faces the mucosa (e.g. in the same direction as the mucoadhesive) and/or part of the mucoadhesive layer overlapping the nanoscaffold is adapted to adhesively fix (adhere) the mucoadhesive carrier to the target mucosa.

In a preferred use of the invention the multi-layers of the mucoadhesive patch are precasted followed by loading of the nanofiber reservoir layer with the bacterial particles and antigens. For this purpose, the bacterial particles and antigens are applied as a solution, colloid or suspension onto the nanoscaffold reservoir layer. Uptake by the reservoir layer occurs by diffusion and absorbtion. Excess liquid can be removed by conventional drying or lyophilisation techniques. Inclusion of cryoprotective agent(s) may be required to adequately protect bacterial particles and antigens.

The terms "treatment", "treating", and the like, as used herein include amelioration or elimination of a developed mental disease or condition once it has been established or alleviation of the characteristic symptoms of such disease or condition. As used herein these terms also encompass, depending on the condition of the patient, preventing the onset of a disease or condition or of symptoms associated with a disease or condition, including reducing the severity of a disease or condition or symptoms associated therewith prior to affliction with said disease or condition. Such prevention or reduction prior to affliction refers to administration of the compound or composition of the invention to a patient that is not at the time of administration afflicted with the disease or condition. "Preventing" also encompasses preventing the recurrence or relapse-prevention of a disease or condition or of symptoms associated therewith, for instance after a period of improvement. It should be clear that mental conditions may be responsible for physical complaints. In this respect, the term "treating" also includes prevention of a physical disease or condition or amelioration or elimination of the developed physical disease or condition once it has been established or alleviation of the characteristic symptoms of such conditions.

As used herein, the term "medicament" also encompasses the terms "drug", "therapeutic", or other terms which are used in the field of medicine to indicate a preparation with therapeutic effect.

It will be appreciated that the antigen present in the invention is delivered in a therapeutically effective amount. As used herein, the term "therapeutically effective amount" is meant to refer to an amount of a compound or composition of the present invention that will elicit a desired therapeutic or prophylactic effect or response when administered according to the desired treatment regimen. It is observed that when the immune-dominant antigen is continuously present, the inflammatory antigen-specific cell response is even reduced further. This reduction is significantly larger compared to administration of the antigen as such, the bacterial particles as such, or the noncontinuous presence of the antigen.

Preferably the compound or composition is provided in a unit dosage form, for example a mucoadhesive patch, biofilm or hydrogel is administered to the oral cavity of a subject, e.g. a patient.

The active ingredients may be administered from 1 to 6 times a day, sufficient to exhibit the desired activity. These daily doses can be given as a single dose once daily, or can be given as two or more smaller doses at the same or different times of the day which in total give the specified daily dose. Preferably, the active ingredient is administered once or twice a day. For instance, one dose could be taken in the morning and one later in the day.

In all aspects of the invention, the daily maintenance dose can be given for a period clinically desirable in the patient, for example from 1 day up to several years (e.g. for the mammal's entire remaining life); for example from about (2 or 3 or 5 days, 1 or 2 weeks, or 1 month) upwards and/or for example up to about (5 years, 1 year, 6 months, 1 month, 1 week, or 3 or 5 days). Administration of the daily maintenance dose for about 3 to about 5 days or for about 1 week to about 1 year is typical. Other constituents of the final formulations may include preservatives, inorganic salts, acids, bases, buffers, nutrients, vitamins, or other pharmaceuticals.

The bacterial particles combined with the antigen may be delivered in a dose of at least 0.1 mg to 60 mg (dry weight) per day, preferably between 0.5 and 20 mg per day, most preferably between 1 and 10 mg per day.

The invention further relates to the following numbered embodiments:
1. A mucoadhesive carrier comprising a non-living bacterial particle for use in oral mucosal administration to treat an immune response related disease in a patient, wherein the non-living bacterial particle is combined with at least one antigen causing said immune response, and wherein said immune response related disease is chosen from the group consisting of celiac disease, allergic asthma, allergic rhinitis, allergic conjunctivitis, atopic dermatitis, multiple sclerosis, type I diabetes, autoimmune uveitis, autoimmune thyroiditis, autoimmune myasthenia gravis, rheumatoid arthritis, pemphigus vulgaris or food allergy.
2. A mucoadhesive carrier according to embodiment 1, wherein the carrier is a mucoadhesive patch, biofilm or hydrogel.
3. A mucoadhesive patch according to embodiment 2, wherein said patch comprises electrospun fibers.
4. A mucoadhesive carrier according to any of embodiments 1 to 3, wherein said antigen is α-gliadin or hordein.
5. A mucoadhesive carrier according to any of embodiments 1 to 3, wherein said antigen is one ore more immunodominant epitopes of house dust mite, preferably Der p 2.1.
6. A mucoadhesive carrier according to any of embodiments 1 to 3, wherein said antigen is one ore more immunodominant epitopes of peptides from cat saliva, skin or glands, preferably Fel d 1.
7. A mucoadhesive carrier according to any of embodiments 1 to 3, wherein said antigen is one ore more immunodominant epitopes of peptides involved in Type 1 Diabetes, preferably pINS, GAD65, InsB₉₋₂₃ or IA2.
8. A non-living bacterial particle for use in oral mucosal administration to treat an immune response related disease in a patient, wherein the non-living particle is combined with at least one antigen causing said immune response, and wherein said immune response related disease is chosen from the group consisting of celiac disease, allergic asthma, allergic rhinitis, allergic conjunctivitis, atopic dermatitis, multiple sclerosis, type I diabetes, autoimmune uveitis, autoimmune thyroiditis, autoimmune myasthenia gravis, rheumatoid arthritis, pemphigus vulgaris or food allergy.
9. A mucoadhesive carrier according to any of embodiments 1 to 7, or a non-living bacterial particle according to embodiment 8, wherein the non-living bacterial particle is an acidic heat treated Gram-positive bacterium, preferably *L. lactis.*
10. A mucoadhesive carrier according to any of embodiments 1 to 7, or a non-living bacterial particle according to embodiment 8, wherein the non-living bacterial particle is an empty bacterial envelope obtained from lysing a Gram-negative bacterium, preferably *E. coli* Nissle.
11. A mucoadhesive carrier according to any of embodiments 1 to 7, or a non-living bacterial particle according to embodiment 8, wherein the non-living bacterial particle is a chemically sterilized bacterium, preferably *L. lactis, Lb. rhamnosus, or E. coli* Nissle.
12. A mucoadhesive carrier or non-living bacterial particle according to any of embodiments 1 to 11 for use as a medicament to treat humans and/or animals.

In this document and in its claims, the verb "to comprise" and its conjugations is used in its nonlimiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

The word "about" or "approximately" when used in association with a numerical value (e.g. about 10) preferably means that the value may be the given value (of 10) more or less 0.1% of the value. All patent and literature references cited in the present specification are hereby incorporated by reference in their entirety.

The present invention is further described by the following examples which should not be construed as limiting the scope of the invention.

### EXAMPLES

In one of our studies we have tested a suitable OVA-mCherry mRNA construct. The functionality of the mRNA construct was tested for mCherry expression in vitro using HEK and/or HELA cells. TBPs were covalently labeled with Alexa-647. The fluorescently labeled TBPs mixed with OVA-mCherry mRNA was spotted onto mucoadhesive patches optimally designed in terms of the polymers used for the electrospun nanofibres just prior to buccal administration in piglets. As a control, OVA-mCherry LNPs was spotted onto mucoadhesive patches and taken along in the piglet experiments. The patches were in place for 30-45 min in the piglets. Tissues (mucosal tissue at patch administration site and oral cavity draining lymph nodes) were collected 1, 6 and 24 hrs after patch administration, and wereanalyzed by confocal fluorescence microscopy for the presence of TBP and GFP in tissue samples taken 1 and 6 hrs after administration. OVA-specific tolerance indicatied cytokines IL-10 and TGFβ were analyzed by qPCR in tissue samples taken 24 hrs after patch administration and were showing an excellent immune tolerance profile towards the OVA antigen.

### Example 1 mRNA construct acquisition/production.

DNA sequences coding ovalbumin protein (OVA) and mCherry protein were amplified and cloned into pUC-derived vector for propagation in E. coli cells. This DNA construct contained the T7 promoter and specific 5' and 3' untranslated regions (UTR) for enhanced translation efficiency. Seamless cloning approach was used for all the cloning reactions. The DNA plasmid was then linearized and used as a template for in vitro transcription with T7 polymerase and modified nucleotides (5-methylcytosine and pseudouridine). After the transcription itself, 5' cap (Cap 1) and polyA tail were added to newly synthesized RNA.

After the purification, mRNA integrity was analyzed by gel electrophoresis. The mRNA was stored at -80oC until further use. Part of the mRNA was formulated ad mixed with TBPs in appropriate buffer and stored at -80oC until further use, and part of the mRNA is formulated into lipid nanoparticles (LNPs) with cationic lipid (DOTAP) that binds nucleic acid, helper lipids (DOPE, DOPC) and other constituents (cholesterol, PEG) affecting particle stability and biological compatibility. To achieve optimal and reproducible results, LNPs was formulated using a microfluidic system providing stable conditions (pressure, flow, temperature, mixing ratio). This system mixes the aqueous (mRNA) and organic (lipid mixture) phases with subsequent dilution and stored at 2-8oC until further use. Detailed physical-chemical characterization of nanoparticles was performed prior all in-vitro/in-vivo experiments including size measurement, polydispersity index or zeta potential measurement using dynamic light scattering (DLS).

We observed that suitable amounts of stabilized OVA-mCherry mRNA with and without LNP formulation were obtained.

### Example 2. mRNA in vitro expression.

Functionality of the constructed mRNA was tested by evaluating the mCherry expression after transfection of the OVA-mCherry mRNA into human cell lines (HeLa cells or HEK293) to demonstrate the functionality of the mRNA construct. Cells was seeded to confocal microscopy-compatible plates in DMEM medium with FBS, antibiotics and amino acids. mRNA mixed with commercial mRNA transfection reagent (jetMessenger) in mRNA buffer in 1:2 ratio (mRNA/transfection reagent) was added to cells (in approx. 70 % confluency) in medium in a 1:10 volume ratio. mRNA+TBP was used with or without transfection reagent and LNP-mRNA is used without transfection reagent. After 24-48 hour incubation, cells was inspected using fluorescence confocal microscopy for confirming production of fluorescent mCherry fusion protein.

We found that mRNA expression of the WP1 construct was demonstrated in HEK and HELA cells.

### Example 3. Buccal administration of mRNA+TBP using mucoadhesive patches and tissue analysis.

Four to eight week old piglets were used for buccal administration of 2 cm2 mucoadhesive patches to which 10 µL of a mixture of mRNA+TBP were loaded on the patch to obtain 5 µg mRNA + 250 µg TBP, just prior to patch administration in the animals. Patches to which an equal amount of mRNA were loaded in the form of LNP-mRNA, were used as comparators. Patches were administered for 30-45 min during which the animals remain anesthetized. After this period, animals were allowed to recover and to drink and eat ad libitum. The animals were terminated for tissue sample collection (mucosal surface at patch administration site and oral draining lymph nodes) 1 hr, 6 hrs or 24 hrs after patch administration.

The study groups are (2 animals per group):
1. mRNA+TBP, tissue sampling 1 hr post patch administration
2. mRNA+TBP, tissue sampling 6 hr post patch administration
3. mRNA+TBP, tissue sampling 24 hr post patch administration
4. LNP-mRNA, tissue sampling 1 hr post patch administration
5. LNP-mRNA, tissue sampling 6 hr post patch administration
6. LNP-mRNA, tissue sampling 24 hr post patch administration

### A. TBP and mCherry presence in piglet tissues.

Excised tissue samples were immediately frozen using n-heptane (-80°C). 15µm cross-section tissue slides will be prepared using Cryocut (Leica). Samples for confocal microscopy analysis was stained using Hoechst fluorescent dye (nuclei staining) and observed using confocal microscope Leica SP8 equipped with white laser allowing the selection of appropriate wavelengths, and sensitive hybrid detectors.

As an outcome of this study we have detected and co-locolized:
- mCherry in mucosal tissues
- TBP in mucosal tissues
- mCherry in oral draining lymph nodes
- TBP in oral draining lymph nodes
- mCherry and TBP in mucosal tissues
- mCherry and TBP in oral draining lymph nodes.

### B. Analysis of OVA-specific tolerance biomarkers in piglet tissues.

From excised oral draining lymph nodes single-cell suspensions will be prepared and CD4+ T cells will be isolated and sorted, followed by qPCR detection of IL-10 and TGFβ.

We have observed detection of OVA-specific CD4+ T cells that produce IL-10 and TGFβ, proofing that an excellent immune toleragenic effect has been obtained with the mRNA molecule encoding the OVA antigen.

## Claims

**1.** A mucoadhesive carrier comprising a non-living bacterial particle and a mRNA molecule encoding an antigen for use in inducing immune tolerance to the antigen in a subject, wherein the mucoadhesive carrier is mucosally administered to the subject.

**2.** A mucoadhesive carrier for a use according to claim 1, wherein the antigen causes or is associated with an immune response related disease.

**3.** A mucoadhesive carrier for a use according to claim 2, wherein the immune response related disease is chosen from the group consisting of celiac disease, allergic asthma, allergic rhinitis, allergic conjunctivitis, food allergy, atopic dermatitis, multiple sclerosis, type I diabetes, autoimmune uveitis, autoimmune thyroiditis, autoimmune myasthenia gravis, rheumatoid arthritis, pemphigus vulgaris, Sjögren's disease, Neuromyelitis Optica, Parkinson's Disease.

**4.** A mucoadhesive carrier for a use according to any one of claims 1 - 3, wherein the antigen is selected from the group constisting of α-gliadin, hordein, an immunodominant epitope of house dust mite, an immunodominant epitopes of peptides from cat saliva, skin or glands, and an immunodominant epitopes of peptides involved in Type 1 Diabetes

**6.** A mucoadhesive carrier for a use according to any one of the preceding claims, wherein the mucoadhesive carrier is administered to the oral mucosa.

**7.** A mucoadhesive carrier for a use according to any one of the preceding claims, wherein the carrier is a mucoadhesive patch, biofilm or hydrogel.

**8.** A mucoadhesive patch for a use according to claim 7, wherein said patch comprises electrospun fibers.

**9.** A mucoadhesive patch for a use according to claim 7, wherein the electrospun fibers are made of one or a combination of two or more of the polymers from the list of: Polyethylene Glycol (PEG), Poly(lactic-co-glycolic acid) (PLGA), Polycaprolactone (PCL), Polyvinyl Alcohol (PVA), Chitosan, Poly(lactic acid) (PLA), Polyethyleneimine (PEI) and Gelatin.

**10.** A mucoadhesive carrier for a use according to any one of the preceding claims, wherein the non-living bacterial particle is derived from a probiotic bacterium.

**10.** A mucoadhesive patch for a use according to claim 9, wherein the probiotic bacterium is a *Lactobacillius,* a *Lactococcus,* a *Bifidobacterium* or an *Escherichia coli* Nissle.

**11.** A mucoadhesive patch for a use according to claim 9 or 10, wherein the non-living bacterial particle is selected from:
a) a heat treated Gram-positive bacterium, preferably *L. lactis*;
b) an empty bacterial envelope obtained from lysing a Gram-negative bacterium, preferably *E. coli* Nissle; or,
c) a sterilized bacterium, preferably *L. lactis, Lb. rhamnosus,* or *E. coli* Nissle.

**12.** A mucoadhesive carrier according to any one of the preceding claims for use as a medicament to treat humans and/or animals.
